(19) Europäisches Patentamt
European Patent Office
Office européen des brevets

(11) Publication number: **0 216 351 B1**

# (12) EUROPEAN PATENT SPECIFICATION

(45) Date of publication of patent specification: **02.12.92**

(51) Int. Cl.[5]: **C07C 46/10**, C07C 50/04

(21) Application number: **86113065.6**

(22) Date of filing: **23.09.86**

The file contains technical information submitted after the application was filed and not included in this specification

## (54) Process for purification of oxidation reaction mixture.

(30) Priority: **24.09.85 JP 208949/85**

(43) Date of publication of application:
**01.04.87 Bulletin 87/14**

(45) Publication of the grant of the patent:
**02.12.92 Bulletin 92/49**

(84) Designated Contracting States:
**CH DE FR LI**

(56) References cited:
**DE-A- 2 655 082**
**US-A- 3 625 983**

(73) Proprietor: **MITSUBISHI GAS CHEMICAL COMPANY, INC.**
**5-2, Marunouchi 2-chome Chiyoda-Ku**
**Tokyo, 100(JP)**

(72) Inventor: **Yui, Tomoyuki**
**22-1, 3-chome Miyazono**
**Nagareyama-shi Chiba-ken(JP)**
Inventor: **Hagiwara, Isao**
**No. 14-6, Jinyamae Tokiwadaira**
**Matsudo-shi Chiba-ken(JP)**

(74) Representative: **Türk, Gille, Hrabal, Leifert**
**Brucknerstrasse 20**
**W-4000 Düsseldorf 13(DE)**

EP 0 216 351 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### 1. FIELD OF THE INVENTION

The present invention relates to a process for purifying an oxidation reaction mixture, and more particularly to a process for purifying an oxidation reaction mixture containing 2,3,5-trimethylbenzoquinone (hereinafter abbreviated to "TMBQ")resulting from oxidation of 2,3,6-trimethylphenol (hereinafter abbreviated to "TMP") with oxygen gas or oxygen-containing gas in the presence of a copper chloride complex catalyst.

TMBQ is useful as an intermediate substance for the preparation of Vitamin E.

### 2. DESCRIPTION OF THE PRIOR ART

From US-A-3 625 983 it is already known that 2,6-di-tertalkyl-p-benzoquinone can be prepared by oxidation of 2,4,6-trialkyl-phenol. In JP-A-100 624/83, 137 710/84 and 110 419/84 it is already disclosed that TMBQ can be prepared by oxidation of TMP with oxygen gas or oxygen-containing gas in the presence of a copper chloride complex catalyst.

In the oxidation of TMP with oxygen gas or oxygen-containing gas in the presence of a copper chloride-complex catalyst, chlorinated compounds of TMP, TMBQ and oxidation solvent are formed as by-products. A part of these chlorinated compounds decompose with the generation of hydrochloric acid in processes subsequent to the oxidation reaction. Thus the chlorinated compounds cause corrosion of construction material, accelerate decomposition of TMBQ, or become poisons for a catalyst in the hydrogenation of TMBQ for the production of 2,3,5-trimethylhydroquinone (hereinafter abbreviated to "TMHQ"). Accordingly it is necessary to make the oxidation reaction mixture free of such chlorinated compounds to an extent as high as possible.

It has been found that in the oxidation of TMP in an organic solvent by the use of a copper chloride complex catalyst, chlorinated compounds having the following formulae and other unidentified chlorinated compounds are formed:

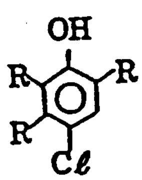 , 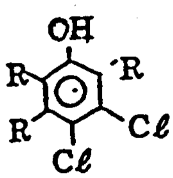 , 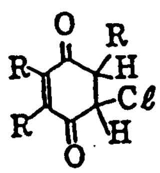 , 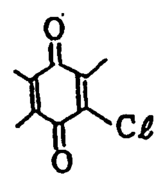 ,

wherein R represents a methyl group. Although the amount of each chlorinated compound formed is not clear bacause its quantitative analysis is quite difficult, the total chlorine content is evaluated as 0,1 to 0,2 wt% in oxidation reaction solution. The chlorinated compounds exert adverse influence on the distillation process of TMBQ or the hydrogenation process of TMBQ to TMHQ.

That is, in the oxidation of TMP by the use of a copper chloride complex catalyst, TMBQ formed is separated from the oxidation reaction mixture by techniques such as dehydration distillation and solvent recovery distillation. At any of these operations, however, a part of chlorinated compounds decompose, thereby generating hydrochloric acid, which will become a catalyst for decomposition of TMBQ in a distillation column and corrode the distillation column. In particular, the corrosion of the solvent recovery distillation column becomes vigorous because the chlorinated compounds are concentrated therein. Furthermore, a part of the chlorinated compounds become poisons for a catalyst used in the hydrogenation of TMBQ.

Thus it is quite important to remove the chlorinated compounds from the oxidation reaction mixture. However, all the chlorinated compounds contained in the oxidation reaction mixture do not decompose at the subsequent steps or become poisons for the hydrogenation catalyst. It suffices, therefore, that the more active chlorinated compounds are removed from the oxidation reaction mixture.

### 3. SUMMARY OF THE INVENTION

Therefore, the object of the present invention is to provide a process for removal of chlorinated compounds from the oxidation reaction mixture which compounds are responsible for the corrosion of the

construction material, the decomposition of TMBQ or become catalyst poisons for a hydrogenation catalyst in the hydrogenation of TMBQ to TMHQ.

It has been found that active chlorinated compounds can be removed by treating the oxidation reaction mixture at an elevated temperature with an aqueous alkali solution, so that the decomposition of TMBQ during the distillation process, the corrosion of a distillation column, and the degradation of a hydrogenation catalyst can be substantially prevented.

Subject-matter of the present invention is a process for purifying an oxidation reaction mixture resulting from oxidation of 2,3,6-trimethylphenol (TMP) with oxygen or oxygen-containing gas in the presence of a copper chloride complex catalyst, which process comprises treating the oxidation reaction mixture at a temperature of from 120 to 150 ° C with an aqueous alkali solution to remove chlorinated compounds contained in the oxidation reaction mixture as by products.

Preferred embodiments of the above process of the invention are described in subclaims 2 to 11.

## 4. DETAILED DESCRIPTION OF THE INVENTION

Useful alkalis for the aqueous alkali aolution which is used in the process of the present invention are, for example, sodium hydroxide, potassi m hydroxide, calcium hydroxide (milk of lime), and ammonia. In particular, aqueous solutions of sodium hydroxide and potassium hydroxide are preferred from the viewpoint of performance of chlorine removal and selectivity.

The concentration of the alkali in the aqueous alkali solution is usually 0.1 to 10 wt%, preferably 0.1 to 5 wt% and more preferably 0.1 to 0:5 wt% although the optimum concentration varies slightly with the type of the alkali used. If the concentration of the aqueous alkali solution is too high, the concentration of the alkali in the treating vessel is liable to be uneven. In this case, to make the concentration of the alkali uniform throughout the treating vessel, a large agitation power is needed. If the agitation is not sufficient, there is a large possibility of a high concentration of the alkali coming into contact with TMBQ, thereby causing decomposition of the TMBQ.

Treatment of the oxidation reaction mixture with the aqueous alkali solution can be achieved by merely mixing and stirring them. For efficient removal of the chlorinated compounds, it is necessary to properly choose the treatment temperature, pH and the amount of water used. TMBQ relatively easily decomposes in an alkaline environment. This tendency is promoted as the temperature rises. Thus, conditions of treatment for the removal of the chlorinated compounds should be determined taking into consideration also the decomposition of TMBQ.

In connection with the pH as one of the conditions of treatment, as the pH is lower, the reaction between the chlorinated compounds and the alkali is increasingly retarded and, as a result, the treatment time is undesirably lengthened. If the pH is more than 10, the decomposition of TMBQ undesirably occurs to a considerable extent. That is, if the pH is high, the extent of removal of the chlorinated compounds is increased, but the major portion of the alkali added may be consumed in the decomposition of TMBQ, undesirably leading to a decrease in the so-called selectivity of removal of the chlorinated compounds.

Accordingly, the pH range is from 4 to 10, preferably 5-10, and in practice, it is determined by considering the treatment temperature.

In connection with the treatment temperature as another condition of the treatment, as the temperature is higher, the chlorinated compounds are more efficiently removed, but the treatment temperature greatly influences the decomposition of TMBQ. That is, since TMBQ considerably decomposes at 160°C, it is necessary to carry out the treatment at temperatures lower than 160°C. The temperature range is from 120 to 150°C, and in practice, it is determined taking into account the pH value.

Another condition for the treatment is the amount of water used in relation to the amount of the oxidation reaction mixture. As the amount of water used is increased, the extent of removal of the chlorinated compounds is increased. However, the amount of waste water of course is also increased, which is undesirable from an economic standpoint. On the other hand, if the amount of water used is decreased, separation of an organic layer from an aqueous layer becomes difficult. Thus the amount of water used is determined taking into account the extent of removal of the chlorinated compounds, the amount of waste water, and the ease of operation. The amount of water used is such that the water/oxidation reaction mixture is 2/1 to 0.01/1, preferably 1/1 to 0.1/1, and most preferably 1/1 to 0.4/1 in weight ratio.

The process of the present invention can be carried out with both continuous and batchwise procedure. In connection with the removal of chlorine, there is almost no difference between the continuous and batchwise procedure. Even in the case of the continuous process, it is sufficient to use a single treating vessel. The residence time is usually 1 to 3 hours although it varies with conditions such as the treatment temperature and the type of the alkali used. In the case of the batchwise procedure, it suffices that the

treatment time is 0.5 to 2.5 hours.

The copper chloride complex as used herein is a compound in which copper and chloride are coordinated, i.e., a compound represented by the following general formula:

$M_\ell[Cu(II)_mX_n]_p$

wherein M represents an alkali metal belonging to IA in the Periodic Table or ammonium, Cu(II) represents a divalent copper, X represents chloride ℓ represents an integer of 1 to 3, m represents 1 or 2, n represents an integer of 3 to 8, p represents 1 or 2, and 1 + 2mp = np. This compound may or may not contain water of crystallization. In the above general formula, M is an alkali metal or ammonia. The alkali metals include Li, Na, K, Rb and Cs. Of these metals, Li, K and Cs are preferred. Particularly preferred is Li. Representative examples of the copper chloride complex follow:
$Li(CuC\ell_3)\cdot 2H_2O$, $NH_4(CuC\ell_3)\cdot 2H_2O$, $(NH_4)_2(CuC\ell_4)\cdot 2H_2O$, $K(CuC\ell_3)$, $K_2(CuC\ell_4)\cdot 2H_2O$, $Cs(CuC\ell_3)$-$\cdot 2H_2O$, $Cs_2(CuC\ell_4)\cdot 2H_2O$, and $Cs_3(Cu_2C\ell_7)\cdot 2H_2O$.

These copper chloride complexes can be prepared by known methods, such as the methods described in Mellor's Comprehensive Treatment on Inorganic and Theoretical Chemistry, Vol. III, pp. 182-201 (Longman). The copper chloride complexes thus prepared can be identified by measurement of the melting point and measurement of the visible spectrum.

Such copper chloride complexes can be used as catalysts in the oxidation of TMP to TMBQ, e.g. in the manner described in the aforementioned JP-A-100624/1983 and 137710/1984, which are the priority applications under 35 U.S.C. 119 for, respectively, United States Patent Applications Serial Nos. 06/796,485 (CIP of USSN 615,125), filed November 6, 1985 and 06/749,191, filed June 26, 1985. Said United States Patent Applications are hereby incorporated herein by reference. Oxidation reaction mixtures as described therein can be purified utilizing the present invention.

In accordance with the present invention, chlorinated compounds which exert adverse influence in the subsequent steps among those chlorinated compounds present in an oxidation reaction mixture resulting from oxidation of TMP in the presence of a copper chloride complex catalyst, can be removed by treating the oxidation reaction mixture with an aqueous alkali solution. Thus the problems of decomposition of TMBQ, corrosion of construction material, and degradation of a hydrogenation catalyst can be eliminated. This produces advantages that the TMBQ formed can be largely recovered, an anticorrosion material is not needed because the problem of corrosion does not occur and, therefore, the equipment costs can be greatly reduced, and furthermore since the degradation of the hydrogenation catalyst can be greatly reduced, the amount of TMHQ produced per the catalyst is increased and, therefore, the catalyst cost can be decreased.

As described above, the treatment of an oxidation reaction mixture with an aqueous alkali solution according to the present invention is quite effective and produces a great economical advantage.

The present invention is described in greater detail with reference to the following examples.

## EXAMPLES 1 TO 15

As the aqueous alkali solution, an aqueous caustic soda solution was used, and an oxidation reaction mixture was treated by batchwise procedure. This aqueous caustic soda solution was sent by the use of a pump controlled by a pH meter so as to maintain the pH in the treating vessel at a definite value. The treating vessel was a cylindrical stirring vessel equipped with four baffles. Stirring was performed at a rate that the solution was emulsified. As the oxidation reaction mixture to be treated, 500 grams (g) of an oxidation reaction mixture containing about 125 g of TMBQ was used.

The oxidation reaction mixture was obtained by oxidation with an oxygen-containing gas of TMP dissolved in hexanol, using an aqueous copper chloride complex catalyst, and then separating the organic oxidation reaction mixture phase from the aqueous phase.

The extent of removal of chlorine was examined changing the concentration of the aqueous caustic soda solution, the treating time, the treating temperature, and the pH. The chlorine content was measured by a chlorine analyzer. The results are shown in Table 1.

Table 1

| Example No. | Concentration of NaOH (wt%) | Temperature (°C) | pH | Treating Time (h) | Amount of Chlorine before Treatment*1 (x10⁻² mol/kg) | Amount of Chlorine after Treatment*1 (x10⁻² mol/kg) | Extent of Removal of Chlorine (%) |
|---|---|---|---|---|---|---|---|
| 1 | 5.0 | Room temperature | 10 | 20 | 7.62 | 5.37 | 30 |
| 2 | 0.5 | Room temperature | 10 | 20 | 7.62 | 4.84 | 36 |
| 3 | 0.5 | 50 | 10 | 20 | 7.62 | 3.94 | 48 |
| 4 | 0.5 | 80 | 10 | 2.0 | 7.62 | 3.63 | 52 |
| 5 | 0.5 | 80 | 8 | 2.0 | 7.62 | 4.85 | 36 |
| 6 | 0.5 | Boiling point *2 | 8 | 2.0 | 7.62 | 3.47 | 54 |
| 7 | 0.5 | Boiling point *2 | 8 | 3.0 | 7.62 | 3.26 | 57 |
| 8 | 0.5 | Boiling point *2 | 8 | 20 | 7.62 | 3.09 | 59 |
| 9 | 0.5 | Boiling point *2 | 7 | 2.0 | 7.62 | 3.86 | 49 |
| 10 | 0.5 | 120 | 7 | 3.0 | 7.62 | 1.75 | 77 |
| 11 | 0.5 | 140 | 6 | 3.0 | 7.62 | 1.3 | 83 |
| 12 | 0.5 | 150 | 4 | 3.0 | 7.62 | 1.52 | 80 |
| 13 | 0.5 | 150 | 5 | 3.0 | 7.62 | 1.14 | 85 |
| 14 | 0.5 | 150 | 6 | 3.0 | 7.62 | 0.76 | 90 |
| 15*3 | 0.5 | 150 | 7 | 3.0 | 7.62 | 0.53 | 93 |

*1 Calculated as mol of chlorine per kilogram of TMBQ

*2 95°C

*3 2% of decomposition of TMBQ

EXAMPLES 16 TO 20

The procedures of Examples 1 to 15 were performed by the continuous flow process. The amount of the oxidation reaction mixture to be held in the treating vessel was set at 500 ml and the oxidation reaction mixture was sent to the treating vessel by the use of a quantitative pump in such a manner that a predetermined residence time could be obtained. At the same time, an aqueous alkali solution was introduced into the treating vessel through an alkali feeding pump controlled by a pH controller. The treated solution was continuously withdrawn by overflowing. The results are shown in Table 2.

Table 2

| Example No. | Concentration of NaOH (wt%) | Temperature (°C) | pH | Residence Time (h) | Amount of Chlorine before Treatment*1 ($\times 10^{-2}$ mol/kg) | Amount of Chlorine after Treatment*1 ($\times 10^{-2}$ mol/kg) | Amount of NaOH added *1 ($\times 10^{-2}$ mol/kg) | Extent of Removal of Chlorine (%) |
|---|---|---|---|---|---|---|---|---|
| 16 | 5 | Room temperature | 10 | 0.5 | 6.93 | 5.64 | 3.20 | 19 |
| 17 | 0.5 | Boiling point *2 | 8 | 2.0 | 7.62 | 4.50 | 4.00 | 41 |
| 18 | 0.5 | Boiling point *2 | 8 | 3.0 | 7.62 | 3.96 | 4.55 | 48 |
| 19 | 0.5 | Boiling point *2 | 8.6 | 2.0 | 7.62 | 4.09 | 5.30 | 46 |
| 20 | 0.5 | Boiling point *2 | 9.5 | 2.0 | 7.62 | 3.72 | 8.0 | 51 |

*1 Calculated as mol of chlorine or NaOH per kilogram of TMBQ

*2 95°C

EXAMPLES 21 TO 23

The procedure of Example 18 was repeated wherein the 0.5% aqueous caustic soda solution was replaced with a 0.5% aqueous potassium hydroxide solution; ammonia water, or a slurry of calcium hydroxide. The results are shown in Table 3.

Table 3

| Example No. | Type of alkali | Temperature (°C) | pH | Residence Time (h) | Amount of Chlorine before Treatment*1 ($\times 10^{-2}$ mol/kg) | Amount of Chlorine after Treatment*1 ($\times 10^{-2}$ mol/kg) | Amount of alkali added *1 ($\times 10^{-2}$ mol/kg) | Extent of Removal of Chlorine (%) |
|---|---|---|---|---|---|---|---|---|
| 21 | KOH | Boiling point *2 | 8 | 3 | 7.62 | 4.05 | 4.60 | 47 |
| 22 | $NH_4OH$ | Boiling point *2 | 7.5 | 3 | 7.62 | 4.72 | 4.93 | 38 |
| 23 | $Ca(OH)_2$ | Boiling point *2 | 8 | 3 | 7.62 | 4.55 | 4.25 | 40 |

*1 Calculated as mol of chlorine or alkali per kilogram of TMBQ

*2 95°C

EXAMPLES 24 to 26

The influence of amount of water used were examined in the same manner as in Example 1 except the treating temperature was 150°C, the treating time was 3 hours, and pH was 5. The results are shown in Table 4.

Table 4

| Example No. | Water/Oxidation Reaction Mixture (wt ratio) | Amount of Chlorine before Treatment (x $10^{-2}$ mol/kg•bq) | Amount of Chlorine after Treatment ( x $10^{-2}$ (mol/kg•bq) | Extent of Removal of Chlorine (%) |
|---|---|---|---|---|
| 24 | 0.49 | 9.5 | 2.0 | 79 |
| 25 | 0.72 | 9.5 | 0.8 | 91 |
| 26 | 1.0 | 9.5 | 0 | 100 |

REFERENCE EXAMPLES 1 TO 4

The Oxidation reaction mixtures, untreated or treated with the aqueous alkali solution, in Examples 1, 17 and 18 were dehydrated by distillation. From the top of the distillation column, a water/oxidation reaction solvent azeotrope was obtained. The pH and chlorine content in the water from the top of the distillation column were measured. This chlorine content was measured by the silver nitrate titration method. The results are shown in Table 5.

Table 5

| Reference Example | Oxidation Reaction Mixture to be distilled | pH | Chlorine Content (ppm) |
|---|---|---|---|
| 1 | Example 1 | 3.7 | 11 |
| 2 | Example 17 | 5.2 | 0 |
| 3 | Example 18 | 5.6 | 0 |
| 4 | Untreated | 2.7 | 70 |

It can be seen from the results of Table 5 that almost all inorganic chlorine compounds are removed by the treatment with the aqueous alkali solution. In distillation of the untreated oxidation reaction mixture, a chloric acid-resistant construction material is needed. On the other hand, the oxidation reaction mixture treated with the aqueous alkali solution does not need any chloric acid-resistant material in its distillation.

REFERENCE EXAMPLES 5 TO 8

The oxidation reaction mixtures, untreated or treated, in Examples 1, 17 and 18 were heated at 115°C at which the distillation column was operated to examine the rate of decomposition of TMBQ. Analysis of TMBQ was performed by gas chromatography.

Table 6

| Reference Example | Type of Oxidation Reaction Mixture | Rate of Decomposition of TMBQ (%/h) |
|---|---|---|
| 5 | Example 1 | 0.2 |
| 6 | Example 17 | 0 |
| 7 | Example 18 | 0 |
| 8 | Untreated | 1.8 |

REFERENCE EXAMPLES 9 TO 15

A corrosion test for the construction material was performed for an oxidation reaction mixture treated or untreated with an aqueous alkali solution, at a temperature of 115°C at which the dehydration and solvent recovery distillation were carried out. The test piece used was a welded piece cut out in a circular form having a diameter of 15 mm. Each test was run for a period of 200 hours.

The piece for the corrosion test was prepared by the usual procedure comprising abrasion using an emery abrasive paper, degreasing with acetone, and drying. The results are shown in Table 7.

Table 7

| Reference Example | Type of the Oxidation Reaction Mixture | Dehydration Column | | | |
|---|---|---|---|---|---|
| | | SUS 304 | | SUS 316 | |
| | | Change of Appearance | Decrease in weight (g) | Change of Appearance | Decrease in weight (g) |
| 9 | Example 1 | Slight discoloration | 0 | No discoloration | 0 |
| 10 | Example 20 | No discoloration | 0 | do | 0 |
| 11 | Example 21 | do | 0 | do | 0 |
| 12 | Example 22 | do | 0 | do | 0 |
| 13 | Example 11 | do | 0 | do | 0 |
| 14 | Example 14 | do | 0 | do | 0 |
| 15 | Untreated | Entirely discolored Corrosion | 0.085 | Entirely discolored Corrosion | 0.0305 |

Table 7 (Continued)

| Reference Example | Solvent Recovery Column: SUS 304 Change of Appearance | SUS 304 Decrease in Weight (g) | SUS 316 Change of Appearance | SUS 316 Decrease in Weight (g) |
|---|---|---|---|---|
| 9 | Pitting | 0.066 | Pitting | 0.005 |
| 10 | do | 0.07 | do | 0.003 |
| 11 | do | 0.065 | do | 0.003 |
| 12 | do | 0.08 | do | 0.001 |
| 13 | No change | 0 | No change | 0 |
| 14 | do | 0 | do | 0 |
| 15 | Vigorous corrosion | 0.13 | Vigorous corrosion | 0.093 |

## REFERENCE EXAMPLES 16 TO 19

The oxidation reaction mixtures, untreated or treated, in Examples 1, 17 and 18 were distilled to separate TMBQ. TMBQ thus obtained was dissolved in an organic solvent and hydrogenated by the use of an alumina-supported palladium catalyst. This hydrogenation reaction was carried out by batchwise procedure. The hydrogenation reaction was repeated to examine the change in activity of the catalyst. The results are shown in Table 8.

Table 8

| Reference Example | Oxidation Reaction Mixture resulting from TMBQ | Extent of Retention of Catalytic Activity after 30 Bätches *1 (%) |
|---|---|---|
| 16 | Example 1 | 88 |
| 17 | Example 17 | 96 |
| 18 | Example 18 | 94 |
| 19 | Untreated | 5 |

$$*1\quad \frac{\text{Rate of reaction after 30 batches}}{\text{Initial rate of reaction}} \times 100$$

## Claims

1. A process for purifying an oxidation reaction mixture resulting from oxidation of 2,3,6-trimethylphenol with oxygen gas or oxygen-containing gas in the presence of a copper chloride complex catalyst, which process comprises treating the oxidation reaction mixture at a temperature of from 120 to 150°C with an aqueous alkali solution to remove chlorinated compounds contained in the oxidation reaction mixture as by-products.

2. The process of Claim 1, wherein said aqueous alkali solution is an aqueous solution of at least one alkali selected from the group consisting of sodium hydroxide, potassium hydroxide, calcium hydroxide and ammonia.

3. The process of Claim 1 or 2, wherein said aqueous alkali solution is an aqueous solution of sodium hydroxide or potassium hydroxide.

4. The process of any of claims 1 to 3 wherein the concentration of the alkali in the aqueous alkali solution is 0.1 to 10 percent by weight.

5. The process of Claim 4, wherein the concentration of the alkali in the aqueous solution is 0.1 to 0.5 percent by weight.

6. The process of any of claims 1 to 5 wherein the treatment pH is from 4 to 10.

7. The process of any of claims 1 to 6, wherein the amount of water used in relation to the amount of the oxidation reaction mixture is such that the water/oxidation reaction mixture is 2/1 to 0.01/1 in weight ratio.

8. The process of Claim 7, wherein the amount of water used in relation to the amount of the oxidation reaction mixture is such that the water/oxidation reaction mixture is 1/1 to 0.4/1 in weight ratio.

9. The process of any of claims 1 to 8, wherein the treatment time is from 1 to 3 hours.

10. The process of Claim 6, wherein the treatment pH is from 5 to 10.

**11.** The process of any claims 1 to 10, wherein
the concentration of the alkali in the aqueous solution is 0.1 to 0.5 percent by weight;
the treatment pH is from 5 to 10;
the treatment temperature is from 120 to 150°C; and
the amount of water used in relation to the amount of the oxidation reaction mixture is such that the water/oxidation reaction mixture is 1/1 to 0.4/1 in weight ratio.

**Patentansprüche**

**1.** Verfahren zur Reinigung einer Oxidationsreaktionsmischung, die aus der Oxidation von 2,3,6-Trimethylphenol mit Sauerstoffgas oder einem Sauerstoff enthaltenden Gas in Gegenwart eines Kupferchlorid-Komplex-Katalysators resultiert, das umfaßt die Behandlung der Oxidationsreaktionsmischung bei einer Temperatur von 120 bis 150°C mit einer wäßrigen Alkalilösung zur Entfernung der in der Oxidationsreaktionsmischung als Nebenprodukte enthaltenen chlorierten Verbindungen.

**2.** Verfahren nach Anspruch 1, worin die wäßrige Alkalilösung eine wäßrige Lösung mindestens eines Alkalis, ausgewählt aus der Gruppe, die besteht aus Natriumhydroxid, Kaliumhydroxid, Calciumhydroxid und Ammoniak, ist.

**3.** Verfahren nach Anspruch 1 oder 2, worin die wäßrige Alkalilösung eine wäßrige Lösung von Natriumhydroxid oder Kaliumhydroxid ist.

**4.** Verfahren nach einem der Ansprüche 1 bis 3, worin die Konzentration des Alkali in der wäßrigen Alkalilösung 0,1 bis 10 Gew.-% beträgt.

**5.** Verfahren nach Anspruch 4, worin die Konzentration des Alkali in der wäßrigen Lösung 0,1 bis 0,5 Gew.-% beträgt.

**6.** Verfahren nach einem der Ansprüche 1 bis 5, worin der Behandlungs-pH-Wert 4 bis 10 beträgt.

**7.** Verfahren nach einem der Ansprüche 1 bis 6, worin die Menge des verwendeten Wassers, bezogen auf die Menge der Oxidationsreaktionsmischung so ist, daß das Gewichtsverhältnis Wasser/Oxidationsreaktionsmischung 2/1 bis 0,01/1 beträgt.

**8.** Verfahren nach Anspruch 7, worin die Menge des verwendeten Wassers, bezogen auf die Menge der Oxidationsreaktionsmischung so ist, daß das Gewichtsverhältnis Wasser/Oxidationsreaktionsmischung 1/1 bis 0,4/1 beträgt.

**9.** Verfahren nach einem der Ansprüche 1 bis 8, worin die Behandlungszeit 1 bis 3 Stunden beträgt.

**10.** Verfahren nach Anspruch 6, worin der Behandlungs-pH-Wert 5 bis 10 beträgt.

**11.** Verfahren nach einem der Ansprüche 1 bis 10, worin die Konzentration des Alkali in der wäßrigen Lösung 0,1 bis 0,5 Gew.-% beträgt, der Behandlungs-pH-Wert 5 bis 10 beträgt, die Behandlungstemperatur 120 bis 150 ° C beträgt und die verwendete Wassermenge, bezogen auf die Menge der Oxidationsreaktionsmischung, so ist, daß das Gewichtsverhältnis Wasser/Oxidatioflsreaktionsmischung 1/1 bis 0,4/1 beträgt.

**Revendications**

**1.** Procédé pour purifier un mélange réactionnel d'oxydation résultant de l'oxydation du 2,3,6-triméthylphénol par l'oxygène gazeux ou un gaz contenant de l'oxygène en présence d'un catalyseur au complexe de chlorure de cuivre, ce procédé consistant à traiter le mélange réactionnel d'oxydation à une température de 120 à 150 ° C par une solution aqueuse d'alcali pour éliminer les composés chlorés contenus comme sous-produits dans le mélange réactionnel d'oxydation.

**2.** Procédé selon la revendication 1, dans lequel ladite solution aqueuse d'alcali est une solution aqueuse d'au moins un alcali choisi dans le groupe formé par l'hydroxyde de sodium, l'hydroxyde de potassium,

l'hydroxyde de calcium et l'ammoniac.

3. Procédé selon la revendication 1 ou 2, dans lequel ladite solution aqueuse d'alcali est une solution aqueuse d'hydroxyde de sodium ou d'hydroxyde de potassium.

4. Procédé selon l'une quelconque des revendications 1 à 3, dans lequel la concentration de l'alcali dans la solution aqueuse d'alcali est de 0,1 à 10 pour cent en poids.

5. Procédé selon la revendication 4, dans lequel la concentration de l'alcali dans la solution aqueuse est de 0,1 à 0,5 pour cent en poids.

6. Procédé selon l'une quelconque des revendications 1 à 5, dans lequel le pH de traitement est de 4 à 10.

7. Procédé selon l'une quelconque des revendications 1 à 6, dans lequel la quantité d'eau utilisée relativement à la quantité du mélange réactionnel d'oxydation est telle que le rapport en poids de l'eau au mélange réactionnel d'oxydation est de 2/1 à 0,01/1.

8. Procédé selon la revendication 7, dans lequel la quantité d'eau utilisée relativement à la quantité du mélange réactionnel d'oxydation est telle que le rapport en poids de l'eau au mélange réactionnel d'oxydation est de 1/1 à 0,4/1.

9. Procédé selon l'une quelconque des revendications 1 à 8, dans lequel le temps de traitement est de 1 à 3 heures.

10. Procédé selon la revendication 6, dans lequel le pH de traitement est de 5 à 10.

11. Procédé selon l'une quelconque des revendications 1 à 10, dans lequel
la concentration de l'alcali dans la solution aqueuse est de 0,1 à 0,5 pour cent en poids ;
le pH de traitement est de 5 à 10 ;
la température de traitement est de 120 à 150 ° C ; et
la quantité d'eau utilisée relativement à la quantité du mélange réactionnel d'oxydation est telle que le rapport en poids de l'eau au mélange réactionnel d'oxydation est de 1/1 à 0,4/1.